# EUROPEAN PATENT APPLICATION

(11) **EP 3 865 062 A1**
(43) Date of publication of application: **18.08.2021**
(21) Application number: 19871467.7
(22) Date of filing: 06.03.2019
(51) Int. Cl.: A61B 5/06, A61B 1/04, G01R 33/02

(54) **MAGNETIC SOURCE DETECTION DEVICE FIXEDLY CONNECTING EXTERNAL MAGNET AND MAGNETIC SENSOR ARRAY**

(30) Priority: 12.10.2018 CN 201811191675
(71) Applicant: Beijing Institute Of Technology, Beijing 100081 (CN); Huang, Qiang, Beijing 100081 (CN); Li, Jing, Beijing 100081 (CN); Zhou, Jiyang, Beijing 100081 (CN); Paolo, Dario, Beijing 100081 (CN); Gastone, Ciuti, Beijing 100081 (CN); Zhang, Wenhui, Beijing 100039 (CN)
(72) Inventor: HUANG, Qiang, Beijing 100081 (CN); LI, Jing, Beijing 100081 (CN); ZHOU, Jiyang, Beijing 100081 (CN); PAOLO, Dario, Beijing 100081 (CN); GASTONE, Ciuti, Beijing 100081 (CN); ZHANG, Wenhui, Beijing 100039 (CN)
(74) Representative: Vidon Brevets & Stratégie
(86) International application number: PCT/CN2019/000047
(87) International publication number: WO 2020/073540

(57) **Abstract**

The present invention discloses a magnetic source detection device fixedly connected with an external magnet and a magnetic sensor array. The magnetic source detection device includes an external magnet guide module, the external magnet, a connecting module, a data processing module and a detection array composed of magnetic sensors; wherein the external magnet guide module is capable of providing movement in all directions; the connecting module is fixedly connected to the tail end of the external magnet guide module and is used for connecting the magnetic sensors and the external magnet so that the external magnet and the magnetic sensors have fixed relative positions and postures; and the data processing module is used for acquiring magnetic field signals from the magnetic sensors, performing calculation by using a pose detection algorithm and performing data communication with the outside. By using the magnetic source detection device, the magnetic field of the external magnet may be precisely and rapidly removed, and thus, a capsule robot is positioned accurately in real time.

## Description

### TECHNICAL FIELD

The present invention relates to the technical field of real-time detection of mobile magnetic sources in the presence of external magnets, in particular to a magnetic source detection device fixedly connected with an external magnet and a magnetic sensor array.

### BACKGROUND

There are still a lot of difficulties to be solved in practical engineering of an actively guided capsule endoscope system. For example, a capsule is required to be positioned in real time when a capsule robot is guided by an external magnet; however, a strong magnetic field of the external magnet exists in the active capsule robot guided by the external magnet to affect the positioning of the capsule, there are errors when the magnetic field of the external magnet is calculated, it is difficult to realize accurate calculation, meanwhile, there are huge difficulties in real-time positioning, and therefore, it is difficult to position the actual position of the capsule in real time.

### SUMMARY

For this purpose, the prevent invention provides a magnetic source detection device fixedly connected with an external magnet and a magnetic sensor array. By using the device, a magnetic field of the external magnet may be removed precisely and rapidly, and thus, a capsule robot may be positioned accurately in real time.

For solving the above-mentioned technical problems, the present invention is achieved in such a mode:
provided is a magnetic source detection device fixedly connected with an external magnet and a magnetic sensor array, including an external magnet guide module, the external magnet, a connecting module, a data processing module and a detection array composed of magnetic sensors; the external magnet guide module is capable of providing movement in all directions; the connecting module is fixedly connected to the tail end of the external magnet guide module and is used for connecting the magnetic sensors and the external magnet so that the external magnet and the magnetic sensors have fixed relative positions and postures; and
the data processing module is used for acquiring magnetic field signals from the magnetic sensors, performing calculation by using a pose detection algorithm and performing data communication with the outside.

Preferably, the data processing module is mounted at the tail end of the external magnet guide module and includes a processor and communication units;
the communication units are in data communication with the magnetic sensors and the outside of the device in a wired or wireless mode;
the processor mainly executes the following operations:
acquiring the magnetic field signals measured by all the magnetic sensors on the detection array under the conditions that the external magnet is mounted in place and a to-be-detected magnetic source is removed, and subtracting a current geomagnetic signal to obtain a fixed value VI of a magnetic field of the external magnet; and
acquiring the magnetic field signals measured by all the magnetic sensors on the detection array under current poses of the external magnet and the detection array during detection, and subtracting a current geomagnetic signal and the fixed value VI of the magnetic field of the external magnet to obtain an effective magnetic field signal V2 from the to-be-detected magnetic source; and acquiring a pose of the to-be-detected magnetic source relative to the detection array by utilizing the effective magnetic field signal V2.

Preferably, the tail end of the external magnet guide module is further provided with a power source supplying power to the data processing module and the detection array.

Preferably, the connecting module is a disk, the center of the disk is provided with the external magnet, the magnetic sensors are distributed on a circular ring at the outer side of the external magnet, and a circular ring outside the magnetic sensors is provided with the data processing module.

Preferably, all the magnetic sensors in the detection array are mounted according to a distribution direction of the magnetic field of the external magnet, so that a measured value of the magnetic field of the external magnet in at least one measurement direction of the magnetic sensors has a measurement margin not less than 1 Gauss from a measurement range.

Preferably, the measured value of the magnetic field of the external magnet in the at least one measurement direction of the magnetic sensors is close to 0 as far as possible.

Preferably, the magnetic sensors are triaxial magnetic sensors, and coordinate axes of all the magnetic sensors are parallel to each other and are parallel to the coordinate axis of the external magnet; and the magnetic field of the external magnet is perpendicular to a plane composed of XY axes of the magnetic sensors, and at the moment, the measured values of the magnetic field of the external magnet in measurement directions of X axes and Y axes of the magnetic sensors are close to 0.

Preferably, the magnetic sensors are triaxial magnetic sensors, a plane composed of XY axes of each of the magnetic sensors is perpendicular to the magnetic field of the external magnet at the mounting position of the magnetic sensor, and at the moment, the measured values of the magnetic field of the external magnet in measurement directions of X axes and Y axes of the magnetic sensors are close to 0.

Beneficial effects:
according to the present invention, the relative positions and postures of the magnetic sensors and the external magnet are fixed by adopting the connecting module, in this way, a known inherent magnetic field of the external magnet in a detected magnetic source pose detection process may be acquired in advance, then, influences from the magnetic field of the external magnet are effectively reduced and even eliminated in an actual measurement process, and furthermore, an effective magnetic field signal of the capsule robot is acquired to realize high-precision pose detection. Next, due to the relatively fixed connection between the detection array and the external magnet, the detection array and the external magnet move together, so that precise pose information itself may be acquired in real time, and the system complexity may be lowered. In addition, a mode of removing the magnetic field of the external magnet in the present invention may be realized on line, and therefore, real-time properties and rapidity are achieved.

### BRIEF DESCRIPTION OF THE DRAWINGS

Fig. 1 (a) is a vertical view of a device provided by the present invention;
Fig. 1 (b) is a perspective view of the device provided by the present invention;
Fig. 2 is a flow diagram showing an operation process of the device provided by the present invention;
Fig. 3 is a schematic diagram showing a first arrangement mode of a detection array; (a) is a side view, and (b) is a vertical view;
Fig. 4 is a schematic diagram showing a second arrangement mode of the detection array; (a) is a side view, and (b) is a vertical view;
Fig. 5 is a schematic diagram showing a third arrangement mode of the detection array; and
Fig. 6 is a schematic diagram showing current geomagnetism determined according to stored geomagnetic data B1; wherein (a) shows a non-rotation condition; and (b) shows a rotation condition.

Wherein 1, 3-communication unit, 2-microprocessor, 4-power source, 5-magnetic sensor, 6-external magnet, 7-rotating shaft, 8-flange plate, 9-capsule robot, 10-coincident point of stored geomagnetism and sensor chip, 11-non-coincident point of stored geomagnetism and sensor chip, and 12-total magnetic field of certain magnetic sensor chip under rotation condition.

### DETAILED DESCRIPTION

The present invention will be described in detail below in conjunction with the accompanying drawings and embodiments.

In order to accurately position a capsule robot in real time, a magnetic field of an external magnet has to be removed precisely and rapidly. According to such a requirement, the present invention designs a magnetic source detection device fixedly connected with an external magnet and a magnetic sensor array based on a basic thought that relative positions and postures of magnetic sensors and the external magnet are fixed by adopting a connecting module, in this way, a known inherent magnetic field of the external magnet in a detected magnetic source pose detection process may be acquired in advance, then, influences from a magnetic field of the external magnet are effectively reduced and even eliminated in an actual measurement process, and furthermore, an effective magnetic field signal of the capsule robot is acquired to realize high-precision pose detection.

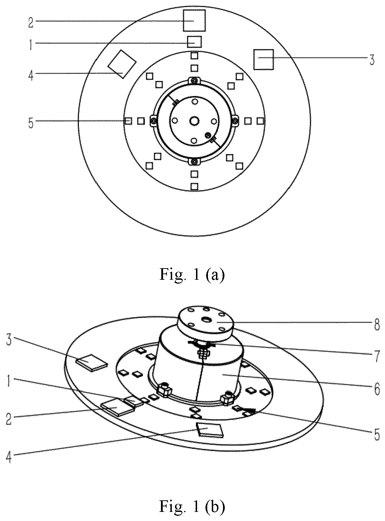

Fig. 1 is an implementation solution of the magnetic source detection device provided by the present invention, and the solution is described with an example in which a to-be-detected magnetic source is a capsule robot 9. As shown in Fig. 1, the magnetic source detection device includes an external magnet guide module, an external magnet 6, a connecting module (a disk shown in the figure), a data processing module and a detection array composed of magnetic sensors 5. Wherein,
only a part of the external magnet guide module is shown in the figure, that is, a flange plate 8 and a rotating shaft 7 (a three-degree-of-freedom translation mechanism is unshown in the figure) capable of rotating at the tail end and relevantly connected are provided. The external magnet guide module is capable of providing movement in six directions for the tail end, and the position and posture of the tail end may be acquired by an internally integrated encoder.

The connecting module is fixedly connected to the tail end of the external magnet guide module. The connecting module is used for connecting the magnetic sensors and the external magnet so that the external magnet and the magnetic sensors have fixed relative positions and postures. The connecting module may be a structural member of any shape as long as the relative positions and postures of the mounted magnetic sensors and external magnet are fixed and known. For example, in the present embodiment, the connecting module is a disk, the center of the disk is provided with the external magnet, the magnetic sensors are distributed on a circular ring at the outer side of the external magnet, and a circular ring outside the magnetic sensors is provided with all devices in the data processing module. For another example, the connecting module includes two disks, wherein the center of the first disk is provided with the external magnet, the magnetic sensors are distributed on a circular ring at the outer side of the external magnet, meanwhile, the second disk is mounted in parallel to the first disk on a plane in an axis direction of the external magnet, the second disk is provided with the data processing module, and units mounted on the two disks are capable of realizing communication through the data processing module.

The external magnet is mounted at the tail end of the external magnet guide module and is used for providing an external driving magnetic field capable of covering the movement range of the capsule robot under the guide of the external magnet guide module.

The data processing module is used for acquiring magnetic field signals from the magnetic sensors, performing calculation by using a pose detection algorithm and performing data communication with the outside. Specifically, the data processing module includes a microprocessor 2, two communication units 1 and 3 and a power source 4. The microprocessor 2 is used for performing calculation, operating an algorithm and processing a communication protocol, etc., is in communication with the plurality of magnetic sensors 5 through the communication unit 1 to receive the magnetic field signals acquired from the magnetic sensors 5, and is then used for operating a pose detection algorithm and transmitting a result to an external control system or computer in a wired or wireless mode provided by the communication unit 3. The communication unit 3 in Fig. 1 performs communication in a wireless mode and takes charge of transmitting the result to an upper computer. The power source is used for supplying power to the overall circuit.

The detection array is composed of the plurality of magnetic sensors 5 which may be uniaxial, biaxial or triaxial. Preferably, the magnetic sensors are mounted according to a distribution direction of the magnetic field of the external magnet, so that a measured value of the magnetic field of the external magnet in at least one measurement direction of the magnetic sensors has a measurement margin not less than 1 Gauss from a measurement range; and preferably, the measured value of the magnetic field of the external magnet in the at least one measurement direction of the magnetic sensors is enabled to be close to 0 as far as possible. In this way, when a relative position of the detection array and the external magnet moves (for example, elastically deforms), no great influences are generated on a detected result, and meanwhile, more convenience is provided for mounting. The detection array provides a detection range capable of covering a movement range of the capsule robot under the guide of the external magnet guide module and meanwhile, realizes pose detection of the capsule robot according to the detected magnetic field signals from the capsule robot.

The present invention provides various arrangement modes of the detection array.

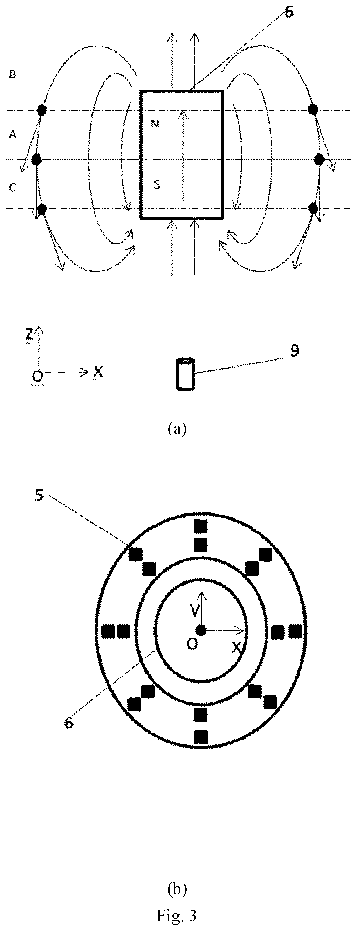

A first mode: triaxial magnetic sensors are adopted and are arranged in a mode as shown in Fig. 3, coordinate axes of all sensor chips are parallel to each other and are parallel to the coordinate axis of the external magnet, and the magnetic sensors are distributed around the external magnet. Optimally, all the magnetic sensors are distributed on a same plane A, at the moment, the magnetic field of the external magnet is perpendicular to a plane composed of XY axes of the magnetic sensors, and the measured values of the magnetic field of the external magnet in measurement directions of X axes and Y axes of the magnetic sensors are close to 0, so that interference from the magnetic field of the external magnet is reduced to the lowest extent.

In practice, the magnetic sensors may also be distributed on a same plane B or C, at the moment, the magnetic field of the external magnet is partially distributed in the X axes and the Y axes of the magnetic sensors, but the distribution of the magnetic field does not exceed the measurement range of the sensors in the measurement direction.

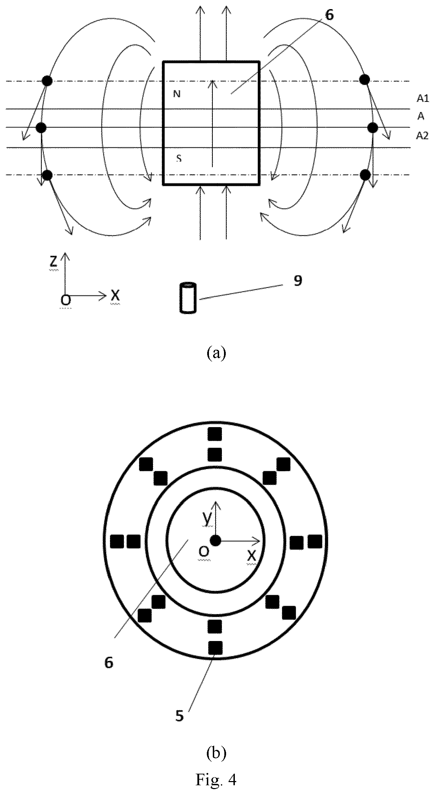

A second mode: triaxial magnetic sensors are adopted and are arranged in a mode as shown in Fig. 4, an arrangement mode of the magnetic sensors is the same as that shown in Fig. 3, that is, coordinate axes of all magnetic sensors are parallel to the coordinate axis of the external magnet, except that the magnetic sensors are not located on the same plane, and sensor chips are spatially distributed. In Fig. 4, the sensor chips are randomly distributed on A, A1 and A2.

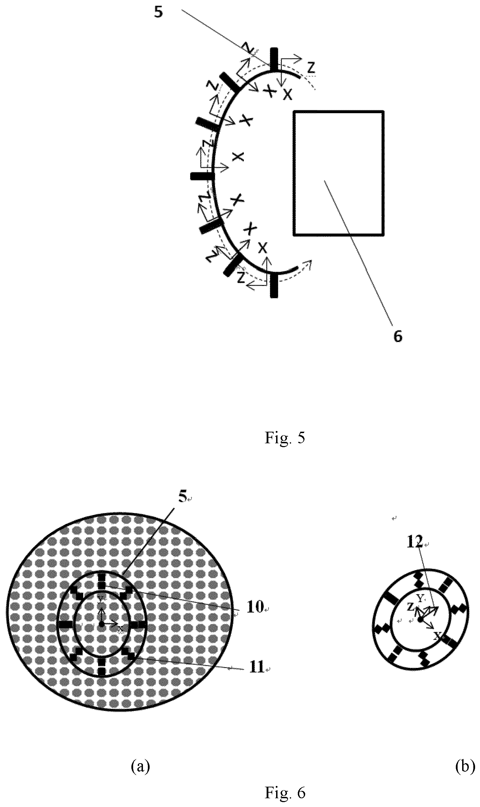

A third mode: triaxial magnetic sensors are adopted and are arranged in a mode as shown in Fig. 5, a plane composed of XY axes of each of the magnetic sensors is perpendicular to the magnetic field of the external magnet on the mounting position of the magnetic sensor, that is, projections of the magnetic field of the external magnet in X-axis and Y-axis directions of the sensor chips are both close to 0, so that the measured values of the magnetic field of the external magnet in measurement directions of X axes and Y axes of the magnetic sensors are close to 0, thereby reducing interference from the magnetic field of the external magnet. The magnetic sensors are distributed around the external magnet and are distributed on the same plane or in a three-dimensional space. The X axis and Z axis of a coordinate system of each of the magnetic sensors are shown as the figure, and the Y axis is perpendicular to a paper surface and is upward.

The detected magnetic source in the present embodiment is the capsule robot. The capsule robot is internally integrated with a camera, a biopsy tool, an internal magnet and the like so as to be capable of completing functions such as endoscope imaging, biopsy and movement driven by an external magnetic field.

A working process of the device provided by the present invention will be described in detail below.

Step 1, the detection array is fixedly connected with the external magnet, and relative poses of the both are kept unchanged. The triaxial magnetic sensors are disposed in the modes as shown in Fig. 3, Fig. 4 or Fig. 5. The overall mounting structure of the data processing module is mounted at the tail end of the external magnet guide module.

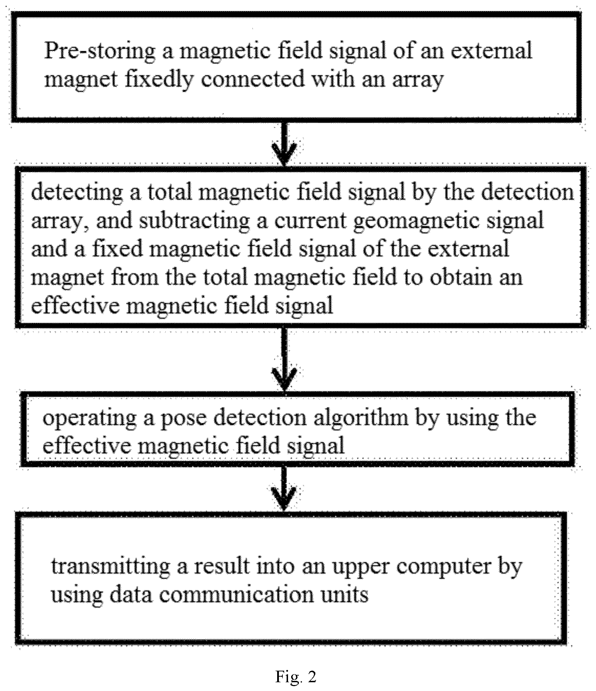

Step 2, after the external magnet and the detection array are mounted, a process is performed as shown in Fig. 2, the relative poses of the external magnet and the magnetic sensors are fixed, therefore, the magnetic field of the external magnet measured by the magnetic sensors is also fixed, and the value of the magnetic field is required to be measured. Specifically, the capsule robot is removed, the microprocessor is adopted to control the magnetic sensors to measure a total magnetic field distributed on the sensors, and at the moment, the total magnetic field includes the magnetic field of the external magnet and a geomagnetic field. The corresponding geomagnetic field is subtracted from the total magnetic field to obtain the simple distribution of the magnetic field of the external magnet on the chips, and the distribution is a fixed value VI of the magnetic field of the external magnet.
BT_EPMx (i)=BT_ALLx (i)-BTl_earthx (i),
BT_EPMy (i)=BT_ALLy (i)-BTl_earthy (i), and
BT_EPMz (i)=BT_ALLz (i)-BTl_earthz (i), wherein
i = 0,1, 2...... Knum-1, and Knum is the number of the triaxial sensors in the array.

BT_ALLx, BT_ALLy and BT_ALLz described herein are triaxial components of a total magnetic field value measured by the triaxial sensors, BT1_ earthx, BTl_earthy and BTl_earthz are triaxial components of a geomagnetic field value at a current position, and BT_EPMx, BT_EPMy and BT_EPMz are fixed distribution, that is, VI, of the magnetic field of the external magnet on the array.

Step 3, in any one detection process, a magnetic field signal in a current environment is subtracted from a total magnetic field signal acquired from the detection array under current poses of the external magnet and the detection array to obtain an effective magnetic field signal V2 from the capsule robot acquired by the detection array, wherein the magnetic field signal in the current environment includes the fixed value V1 (that is, BT_EPMx, BT_EPMy and BT_EPMz) of the magnetic field of the external magnet, a geomagnetic signal at a position where each of the magnetic sensors is located and the like.

Step 4, the microprocessor operates the pose detection algorithm by utilizing the effective magnetic field signal V2 of the capsule robot to obtain pose data of the capsule robot relative to the detection array.

Step 5, the communication units are adopted to transmit the obtained pose data to the upper computer in a wireless mode.

So far, the process is ended.

The acquisition of the geomagnetic signal is mentioned in the above-mentioned step 2 and step 3. The acquisition mode includes the following steps (1) and (2):
(1) A geomagnetic field B1 of each position point within a movement space range of the detection array is pre-stored.
   In practice, the measurement of the above-mentioned geomagnetic field B1 may be completed by adopting additionally disposed magnetic sensors. In order not to increase additional hardware, the acquisition of the geomagnetic field B1 may also be completed by adopting the detection array. Specifically, the external magnet and the capsule robot are removed, one of the magnetic sensors on the detection array is selected, and a coordinate system of the magnetic sensor is enabled to be coincident with a world coordinate system; within the movement space range of the detection array, the detection array is enabled to move in horizontal and vertical directions and uniformly move according to a set density to acquire the magnetic field signal of each position point so as to obtain a geomagnetic field of each position point within the movement space range of the detection array. Herein, the detection array only moves in the horizontal and vertical directions, and therefore, the data storage amount may be reduced. If the calculation of the geomagnetic field of the detection array under a certain rotation pose may be achieved by projecting stored data, the calculation may refer to a second situation in the step (2).
(2) When the current geomagnetic field is used in the step 2 or step 4, the processing is performed in two situations according to the current pose of the detection array and postures of coordinate axes of the sensors when the geomagnetic field B1 is stored:

If the current world coordinate system of the detection array does not rotate relative to the world coordinate system in the pre-storage process in the step (1), the coordinate axis of each of the magnetic sensors is still parallel to the world coordinate system, at the moment, each of the magnetic sensors on the detection array is either coincident with the position of the stored magnetic field or coincident with positions of two or several nearest stored magnetic fields near the magnetic sensor chips. If coincidence such as a coincident point 10 (a great grey point in the figure is a position point of the pre-stored geomagnetic field) in Fig. 6 (a) occurs, the geomagnetic field at the position where each of the magnetic sensors is located is directly extracted from the stored data B1; and if no coincidence such as a non-coincident point 11 in Fig. 4 (a) occurs, all axial magnetic fields of a plurality of magnetic fields on adjacent position points are respectively interpolated to obtain the geomagnetic field at the position where each of the magnetic sensors is located.

If the current world coordinate system of the detection array rotates relative to the world coordinate system in the pre-storage process in the step (1), it is judged whether the position of each of the magnetic sensors on the detection array is coincident with the position of the stored magnetic field after rotation, if coincidence occurs, direct valuing is performed to obtain a magnetic field marked as B1', and if no coincidence occurs, interpolation is performed to obtain a magnetic field marked as B1' which is shown as the total magnetic field at a position point 12 in Fig. 6 (b); and then, the magnetic field B1' is correspondingly projected on the coordinate axis of the detection array under the current pose to obtain a projected triaxial geomagnetic field serving as a current required geomagnetic field signal.

Base on above, the above descriptions are merely preferred embodiments of the present invention, but are not intended to limit the protective scope of the present invention. Any modifications, equivalent replacements, improvements and the like made within the spirit and principle of the present invention shall fall within the protective scope of the present invention.

## Claims

1. A magnetic source detection device fixedly connected with an external magnet and a magnetic sensor array, comprising: an external magnet guide module, the external magnet, a connecting module, a data processing module and a detection array composed of magnetic sensors;
wherein the external magnet guide module is capable of providing movement in all directions; the connecting module is fixedly connected to the tail end of the external magnet guide module and is used for connecting the magnetic sensors and the external magnet so that the external magnet and the magnetic sensors have fixed relative positions and postures; and
the data processing module is used for acquiring magnetic field signals from the magnetic sensors, performing calculation by using a pose detection algorithm and performing data communication with the outside.

2. The device according to claim 1, wherein the data processing module is mounted at the tail end of the external magnet guide module and comprises a processor and communication units;
the communication units are in data communication with the magnetic sensors and the outside of the device in a wired or wireless mode;
the processor mainly executes the following operations:
acquiring the magnetic field signals measured by all the magnetic sensors on the detection array under the conditions that the external magnet is mounted in place and a to-be-detected magnetic source is removed, and subtracting a current geomagnetic signal to obtain a fixed value VI of a magnetic field of the external magnet; and
acquiring the magnetic field signals measured by all the magnetic sensors on the detection array under current poses of the external magnet and the detection array during detection, and subtracting a current geomagnetic signal and the fixed value VI of the magnetic field of the external magnet to obtain an effective magnetic field signal V2 from the to-be-detected magnetic source; and acquiring a pose of the to-be-detected magnetic source relative to the detection array by utilizing the effective magnetic field signal V2.

3. The device according to claim 1, wherein the tail end of the external magnet guide module is further provided with a power source supplying power to the data processing module and the detection array.

4. The device according to claim 1, wherein the connecting module is a disk, the center of the disk is provided with the external magnet, the magnetic sensors are distributed on a circular ring at the outer side of the external magnet, and a circular ring outside the magnetic sensors is provided with the data processing module.

5. The device according to claim 1, wherein all the magnetic sensors in the detection array are mounted according to a distribution direction of the magnetic field of the external magnet, so that a measured value of the magnetic field of the external magnet in at least one measurement direction of the magnetic sensors has a measurement margin not less than 1 Gauss from a measurement range.

6. The device according to claim 5, wherein the measured value of the magnetic field of the external magnet in the at least one measurement direction of the magnetic sensors is close to 0 as far as possible.

7. The device according to claim 6, wherein the magnetic sensors are triaxial magnetic sensors, and coordinate axes of all the magnetic sensors are parallel to each other and are parallel to the coordinate axis of the external magnet; and the magnetic field of the external magnet is perpendicular to a plane composed of XY axes of the magnetic sensors, and at the moment, the measured values of the magnetic field of the external magnet in measurement directions of X axes and Y axes of the magnetic sensors are close to 0.

8. The device according to claim 6, wherein the magnetic sensors are triaxial magnetic sensors, a plane composed of XY axes of each of the magnetic sensors is perpendicular to the magnetic field of the external magnet at the mounting position of the magnetic sensor, and at the moment, the measured values of the magnetic field of the external magnet in measurement directions of X axes and Y axes of the magnetic sensors are close to 0.
